# EUROPÄISCHE PATENTANMELDUNG

(11) **EP 0 571 844 A1**
(43) Veröffentlichungstag der Anmeldung: **01.12.1993**
(21) Anmeldenummer: 93107919.8
(22) Anmeldetag: 14.05.1993
(51) Int. Cl.: C07D 277/72, C07D 277/62

(54) **Verfahren zur Herstellung von 2-Mercaptobenzothiazol und Benzothiazol**

(30) Priorität: 27.05.1992 DE 4217541
(71) Anmelder: BAYER AG, 51368 Leverkusen (DE)
(72) Erfinder: Sicheneder, Adolf, Dr., W-4047 Dormagen 5 (DE); Becker, Bardo, Dr., W-4047 Dormagen (DE)

(57) **Zusammenfassung**

2-Mercaptobenzothiazol und Benzothiazol werden aus Dibenzothiazyldisulfid hergestellt, indem man Dibenzothiazyldisulfid in Gegenwart einer Allkali- und/oder Erdalkalihydroxid-Lösung bei Temperaturen von 40 bis 120°C umsetzt bis sich ein konstanter pH-Wert im Bereich von 8 bis 10 eingestellt hat, dann das Reaktionsgemisch mit einer wäßrigen Lösung von Mineralsäuren bei Temperaturen von 10 bis 100°C behandelt und anschließend Benzothiazol durch Destillation oder Extraktion aus dem 2-Mercaptobenzothiazol enthaltenden Reaktionsgemisch entfernt.

## Beschreibung

Die Erfindung betrifft ein Verfahren zur Herstellung von 2-Mercaptobenzothiazol (MBT) und Benzothiazol (BT) aus Dibenzothiazyldisulfid (MBTS) durch Behandlung des Reaktionsgemisches zunächst mit einer wäßrigen, alkalischen Lösung, danach mit einer wäßrigen Lösung von Mineralsäuren.

Die Herstellung von 2-Mercaptobenzothiazol sowie die Herstellung von Benzothiazol durch Umsetzung von Anilin mit CS₂ und Schwefel ist bekannt (s. US 1.631.871). Je nach Prozeßführung kann man zu einem Gemisch aus MBT/BT gelangen, wobei die Zusammensetzung etwa 10:1 ist. Da bei diesem Herstellungsverfahren nur geringe Mengen an Benzothiazol neben einer größeren Menge an Mercaptobenzothiazol anfällt, ist durch dieses Verfahren die Verfügbarkeit von gewünschtem Benzothiazol limitiert.

Die Herstellung von 2-Mercaptobenzothiazol neben Benzothiazol kann auch durch eine Photolyse von Dibenzothiazyldisulfid erfolgen, wobei allerdings zusätzlich noch Schwefel entsteht (s. Z. Naturforsch., B: Chem. Sci. 1987, 42(9), 1153 bis 1158). Dieses Verfahren ist für eine großtechnische Herstellung von MBT und BT nicht geeignet.

Es wurde nun ein Verfahren zur Herstellung von 2-Mercaptobenzothiazol und Benzothiazol aus Dibenzothiazyldisulfid gefunden, das dadurch gekennzeichnet ist, daß man Dibenzothiazyldisulfid in Gegenwart einer Alkali- und/oder Erdalkalihydroxid-Lösung bei Temperaturen von 40 bis 120°C umsetzt bis sich ein konstanter pH-Wert im Bereich von 8 bis 10 eingestellt hat, dann das Reaktionsgemisch mit einer wäßrigen Lösung von Mineralsäuren bei Temperaturen von 10 bis 100°C behandelt und anschließend das Benzothiazol durch Destillation oder Extraktion aus dem 2-Mercaptobenzothiazol enthaltenden Reaktionsgemisch entfernt.

Das 2-Mercaptobenzothiazol wird aus dem Reaktionsgemisch durch Filtration erhalten.

Die Behandlung des Dibenzothiazyldisulfids mit einer Alkali- und/oder Erdalkalihydroxid-Lösung wird bevorzugt bei Temperaturen von 80 bis 100°C durchgeführt.

Die Konzentration der verwendeten Alkali- und/oder Erdalkalihydroxid-Lösungen beträgt im allgemeinen 10 bis 50 Gew.-%, bevorzugt 20 bis 30 Gew.-%. Als Alkalihydroxide und Erdalkalihydroxide können verwendet werden: LiOH, NaOH, KOH und Ca(OH)₂, bevorzugt NaOH.

Bei dem erfindungsgemäßen Verfahren setzt man pro Mol Dibenzothiazyldisulfid 0,9 bis 1,2 Mol, bevorzugt 1,0 bis 1,1 Mol Alkali- und/oder Erdalkalihydroxid ein.

Die Behandlung des Dibenzothiazyldisulfids mit einer Alkali- und/oder Erdalkalihydroxid-Lösung wird solange fortgesetzt, bis sich ein konstanter pH-Wert eingestellt hat. Zum Ende dieser Behandlung beträgt der pH-Wert etwa 8 bis 10 (Schwankung ± 0.05 Einheiten).

Nachdem der pH-Wert konstant geblieben ist, wird das Reaktionsgemisch mit einer wäßrigen Lösung von Mineralsäuren bei Temperaturen von bevorzugt 20 bis 80°C behandelt. Als Mineralsäuren können eingesetzt werden: Salzsäure, Schwefelsäure, Salpetersäure und/oder Phosphorsäure, bevorzugt Schwefelsäure. Die wäßrigen Lösungen der Mineralsäuren (10 bis 50 %ig) werden in Mengen von etwa 0,7 bis 1,3, bevorzugt 0,9 bis 1,1 Säureäquivalente, bezogen auf eingesetzte Basenäquivalente, eingesetzt. Es ist auch möglich saure Gase, wie HCl oderSO₂, direkt in das wäßrige Reaktionsgemisch einzuleiten.

Bei der Behandlung des Reaktionsgemisches mit der wäßrigen Mineralsäurelösung kann man durch entsprechende Temperaturführung die Zusammensetzung des Produktgemisches regulieren. Höhere Temperaturen (>40°C) begünstigen die Bildung des 2-Mercaptobenzothiazols. Bei tieferen Temperaturen (10 bis 40°C) wird die Bildung von Benzothiazol verstärkt.

Bei dem erfindungsgemäßen Verfahren erhält man eine gute Produktausbeute, wenn man das entstehende Benzothiazol aus dem Reaktionsgemisch ständig entfernt. Dies kann man durch beispielsweise Wasserdampfdestillation (in der Regel bei Normaldruck) oder durch Extraktion mit einem organischen, mit Wasser nicht mischbaren Lösungsmittel, wie Cyclohexan, Hexan, Benzol, Toluol, Xylol, Methyl-tert.-butylether und/oder tert.-Amylmethylether, erreichen.

Weiterhin ist es nach dem erfindungsgemäßen Verfahren möglich durch Variation der Menge an Basen bzw. Säuren die Ausbeute an Benzothiazol und Mercaptobenzothiazol zu beeinflussen. Bei äquimolaren Mengen oder Unterschuß (ca. 10 Mol-%) an Basen erhält man ein Verhältnis von MBT:BT von 3:1. Bei einem Überschuß an Basen (ca. 15 Gew.-%) erhält man im Idealfall die 5-fache molare Menge an 2-Mercaptobenzothiazol.

Das erfindungsgemäße Verfahren ist geeignet zur großtechnischen Herstellung von 2-Mercaptobenzothiazol und Benzothiazol. Die bei dem Verfahren eingesetzte Ausgangsverbindung, das Dibenzothiazyldisulfid, ist ein leicht zugängliches Zwischenprodukt, das bei der Herstellung von Vulkanisationshilfsmitteln eingesetzt wird. Das Dibenzothiazyldisulfid ist auf mehreren Wegen leicht zugänglich, so kann man z.B. 2-Mercaptobenzothiazol mit Sauerstoff und einem geeigneten Katalysatorsystem zum Disulfid umsetzen. Großtechnisch wird bislang Chlor als Oxidationsmittel eingesetzt. Bei der erfindungsgemäßen Herstellung von 2-Mercaptobenzothiazol und Benzothiazol aus Dibenzothiazyldisulfid ist überraschend, daß die Hydrolyse (Disproportionierung) von Dibenzothiazyldisulfid im Alkalischen nicht zur Spaltung des Thiazolsystems führt und die sich anschließende Desulfonierung unter den angegebenen Bedingungen glatt verläuft. Zudem ist überraschend, daß sich das Verhältnis MBT/BT in einem Bereich von 5:1 bis 3:1 durch Variation der Reaktionsparameter reproduzierbar einstellen läßt. Somit erlaubt das Reaktionssystem in gewisser Weise eine bedarfsorientierte Durchführung hinsichtlich der benötigten Produktmengen.

### Beispiele

### Beispiel 1

In einem 500 ml-Vierhalskolben mit KPG-Rührer, Tropftrichter, Thermometer und Rückflußkühler werden
150 Gew.-Teile H₂O
23,2 Gew.-Teile NaOH
vorgelegt und auf 50°C erwärmt. Dann gibt man
85,4 Gew.-Teile Dibenzothiazyldisulfid (MBTS; 98 %ig)
zu und erhitzt auf 90°C. Nach 1 Stunde tropft man innerhalb von etwa 30 Minuten
160 Gew.-Teile H₂SO₄-Lösung (20 %ig)
zu und destilliert dann das entstandene Benzothiazol (BT) azeotrop zu. Im Kolben verbleibt eine Suspension von 2-Mercaptobenzothiazol (MBT) in verdünnter Schwefelsäure. Der Feststoff wird nach Abkühlung abfiltriert und neutral gewaschen. Nach Trocknung erhält man
69,5 Gew.-Teile MBT (96 %ig) und
11,7 Gew.-Teile BT (98 %ig).

Das molare Verhältnis MBT/BT beträgt 4,7:1.

### Beispiel 2

In einem 500 ml-Vierhalskolben mit KPG-Rührer, Tropftrichter, Thermometer und Rückflußkühler legt man
150 Gew.-Teile H₂O und
20,2 Gew.-Teile NaOH
vor und erwärmt auf 50°C. Dann gibt man
85,4 Gew.-Teile MBTS (98 %ig)
zu und erhitzt auf 90°C. Nach einer Stunde tropft man innerhalb von etwa 30 Minuten
160 Gew.-Teile Schwefelsäure (20 %ig)
zu und destilliert dann das entstandene Benzothiazol azeotrop ab. Die zurückbleibende MBT-Suspension wird nach Abkühlung filtriert, der Filterkuchen neutral gewaschen und getrocknet. Man erhält
66,1 Gew.-Teile MBT (96 %ig) und
14,4 Gew.-Teile BT (99 %ig).

Das molare Verhältnis MBT/BT beträgt 3,6:1.

### Beispiel 3

Die Hydrolyse im Alkalischen erfolgt wie in Beispiel 1. Nach einer Stunde Reaktionszeit hat sich ein konstanter pH-Wert eingestellt und man gibt bei 80°C soviel 20 %ige Schwefelsäure zu, daß sich ein pH-Wert von 2 einstellt. Anschließend wird entstehendes Benzothiazol azeotrop abdestilliert. Die zurückbleibende Suspension wird wie in den vorangegangenen Beispielen aufgearbeitet. Man erhält
66,1 Gew.-Teile MBT (95 %ig)
10,9 Gew.-Teile BT (99 %ig).

Das molare Verhältnis MBT/BT beträgt 4,8:1.

### Beispiel 4

In der Apparatur wie unter Beispiel 1 legt man
200 Gew.-Teile H₂O
20,6 Gew.-Teile NaOH
85,4 Gew.-Teile MBTS (98 %ig)
vor und erhitzt die Suspension auf 90°C. Die resultierende Lösung rührt man eine Stunde lang bei dieser Temperatur und tropft dann bei etwa 80°C Schwefelsäure zu, so daß sich ein pH von 4 einstellt. Man verbraucht ungefähr 90 g einer 20 %igen H₂SO₄-Lösung. Zu dieser Suspension gibt man dann
100 Gew.-Teile Cyclohexan
und refluxiert diese Mischung 15 Minuten lang. Anschließend kühlt man ab und filtriert den Feststoff ab und wäscht diesen mit Wasser neutral.

Die organische Phase des Filtrats wird abgetrennt und das Lösungsmittel abdestilliert.

Man erhält
65,6 Gew.-Teile MBT (Gehalt: 98,5 %)
11,4 Gew.-Teile BT (Gehalt: 91,6 %).

Das molare Verhältnis MBT/BT beträgt 5:1.

## Patentansprüche

1. Verfahren zur Herstellung von 2-Mercaptobenzothiazol und Benzothiazol aus Dibenzothiazyldisulfid, dadurch gekennzeichnet, daß man Dibenzothiazyldisulfid in Gegenwart einer Alkali- und/oder Erdalkalihydroxid-Lösung bei Temperaturen von 40 bis 120°C umsetzt bis sich ein konstanter pH-Wert im Bereich von 8 bis 10 eingestellt hat, dann das Reaktionsgemisch mit einer wäßrigen Lösung von Mineralsäuren bei Temperaturen von 10 bis 100°C behandelt und anschließend Benzothiazol durch Destillation oder Extraktion aus dem 2-Mercaptobenzothiazol enthaltenden Reaktionsgemisch entfernt.
